# EUROPEAN PATENT SPECIFICATION

(11) **EP 2 772 219 B1**
(45) Date of publication and mention of the grant of the patent: **06.05.2020**
(21) Application number: 12844181.3
(22) Date of filing: 19.10.2012
(51) Int. Cl.: G16H 40/63, G06Q 50/22

(54) **MEDICAL SYSTEM AND MEDICAL TERMINAL DEVICE**
MEDIZINISCHES SYSTEM UND MEDIZINISCHES ENDGERÄT
SYSTÈME MÉDICAL ET DISPOSITIF DE TERMINAL MÉDICAL

(30) Priority: 25.10.2011 JP 2011233833
(43) Date of publication of application: 03.09.2014
(73) Proprietor: J. Morita Manufacturing Corporation, Kyoto-shi, Kyoto 612-8213 (JP)
(72) Inventor: YOSHIKAWA, Hideki, Kyoto-shi, Kyoto 612-8213 (JP); WADA, Masato, Kyoto-shi, Kyoto 612-8213 (JP); TANAKA, Noriyuki, Kyoto-shi, Kyoto 612-8213 (JP); MURAKAMI, Kuniomi, Kyoto-shi, Kyoto 612-8213 (JP)
(74) Representative: Müller Hoffmann & Partner
(86) International application number: PCT/JP2012/077111
(87) International publication number: WO 2013/061887

(56) References cited:
- WO-A1-2007/016101
- WO-A1-2009/151535
- JP-A- 2003 067 486
- JP-A- 2009 502 337
- US-A1- 2003 097 054

## Description

### TECHNICAL FIELD

The present invention relates a medical terminal device including an interface unit that performs an operation input to a medical care device or the like, and a medical system including such a medical terminal device.

### BACKGROUND ART

Recently, along with rapid advancement of medical care methods, various medical care devices in accordance with such highly advanced medical care methods have been proposed. This results in a site of medical practice having a plurality of operation terminals used to operate a plurality of medical care devices. This may undesirably prevent smooth medical care. However, a medical control device which allows a single operation terminal to operate a plurality of medical care devices has been proposed (see JP-A-2004-532664) .

Such a medical control device allows a single operation terminal (remote control device) to operate a plurality of medical devices as described above, and therefore can reduce the number of operation terminals, which are respectively required for a plurality of medical devices according to the conventional art.

However, such a medical control device has the following problem. When a plurality of operators each perform a medical care on his/her own patient by use of a medical care device corresponding to medical care at the same time, it is difficult to perform a control regarding which operator is using which medical care device for which medical care, namely, which operation terminal is used to operate which medical care device. This may undesirably cause a situation where an operation control is performed on an unintended medical care device.

Document WO 2009/151535 A1 discloses a wireless medical room control arrangement for the control of a plurality of medical devices.

### SUMMARY OF INVENTION

### TECHNICAL PROBLEM

In such a situation, the present invention has an object of providing a medical terminal device that specifies an operator, a patient and a medical care device to be used so that correct medical care can be performed in accordance with the operator and the patient by use of the desired medical care device, and a medical care system including such a medical terminal device.

### SOLUTION TO PROBLEM

the above problem is solved by the subject-matter of the independent claims. The invention is defined by the claims. The examples and embodiments not falling within the scope of the claims are for reference only.

The present invention is directed to a medical system including a medical terminal device including an operation display unit that displays an operation screen used to operate a medical care device, operator identification means that identifies an operator to perform medical care, patient identification means that identifies a patient to receive the medical care, and device identification means that identifies a medical care device to be used for the medical care; a plurality of medical care devices; information storage means that stores device information on the plurality of medical care devices, operator information on a plurality of operators, and patient information on a plurality of patients; and control means that, based on the operator information corresponding to the operator identified by the operator identification means, the patient information corresponding to the patient identified by the patient identification means, and the device information corresponding to the medical care device identified by the device identification means, displays an operation screen on the medical care device to be used for the medical care as an operation screen which reflects a setting made by the operator to perform the medical care and a setting made by the patient to receive the medical care, the operation screen being displayed on the operation display unit.

The medical care device may be, for example, an X-ray imaging device, a medical care unit, a laser treatment device, or a peripheral device such as a root canal length measurement device or the like.

According to the present invention, the operator, the patient, and the medical care device to be used are identified among the plurality of operators, the plurality of patients and the plurality of medical care devices, so that medical care in accordance with the operator and the patient can be correctly performed by use of the desired medical care device.

This will be described in more detail. The medical terminal device includes the operation display unit that displays the operation screen used to operate the medical care device, the operator identification means that identifies the operator to perform the medical care, the patient identification means that identifies the patient to receive the medical care, and the device identification means that identifies the medical care device to be used for the medical care. Therefore, the operator, the patient and the medical care device to be used can be correctly identified and specified among the plurality of operators, the plurality of patients and the plurality of medical care devices.

The medical system further includes the plurality of medical care devices; the information storage means that stores the device information on the plurality of medical care devices, the operator information on the plurality of operators and the patient information on the plurality of patients; and the control means that, based on the operator information corresponding to the operator identified by the operator identification means, the patient information corresponding to the patient identified by the patient identification means and the device information corresponding to the medical care device identified by the device identification means, displays the operation screen regarding the medical care device to be used for the medical care as the operation screen which reflects the setting made by the operator to perform the medical care and the setting made by the patient to receive the medical care, the operation screen being displayed on the operation display unit. Therefore, medical care in accordance with the identified operator and the identified patient can be correctly performed by use of the desired medical care device.

In an embodiment of the present invention, the medical system may further include operator identification information storage means that stores identification information inherent to the operator; patient identification information storage means that stores identification information inherent to the patient; and device identification information storage means that stores identification information inherent to the medical care device. The operator identification means, the patient identification means and the device identification means may perform identification by respectively communicating with the operator identification information storage means, the patient identification information storage means and device identification information storage means.

According to the present invention, the operator, the patient and the medical care device can be identified more correctly, and appropriate medical care can be performed.

This will be described in more detail. The medical system includes the operator identification information storage means that stores the identification information inherent to the operator, the patient identification information storage means that stores the identification information inherent to the patient, and the device identification information storage means that stores the identification information inherent to the medical care device. Therefore, the operator, the patient and the medical care device are identified based on inherent identification information stored on the operator identification information storage means, the patient identification information storage means and the device identification information storage means. Thus, the identification can be performed more correctly.

In an embodiment of the present invention, the plurality of medical care devices may each include terminal device attachment means to which the medical terminal device is attached.

According to the present invention, the medical terminal device capable of operating different types of medical care devices can be attached to the terminal device attachment means of any of these medical care devices. Thus, correct medical care can be performed without using a wrong medical care device.

In an embodiment of the present invention, the operator information may include use permission information on the device which the operator is permitted to use; the medical care device may include use restriction means that provides a switch between permission and restriction on use of the device based on use permission information which permits the device to be used; and the use restriction means may provide a switch between permission and restriction on use of the medical care device identified by the device identification means based on use permission information on the operator identified by the operator identification means.

According to the present invention, based on the use permission information, the medical care device which needs to be used after being permitted to be used is prevented from being used for the medical care by an operator who is not authorized to use the medical care device.

In an embodiment of the present invention, the medical care device and the medical terminal device may each include wireless communication means; and operation information input to the medical terminal device may be transmitted/received via the wireless communication means, so that the medical care device is operated by the medical terminal device.

According to the present invention, the medical care device can be operated by wireless connection without physically connecting the medical care device and the medical terminal device to each other. This improves the convenience.

In an embodiment of the present invention, the patient information may include medical care information on the patient; and the operation display unit may display the medical care information on the patient identified by the patient identification information.

According to the present invention, the medical terminal device can be used to display medical care information which is used to explain the medical care results for informed consent, in addition to being used to operate the medical care device to be used. This also improves the convenience.

In an embodiment of the medical system, the medical care device may include device-side operation means used to operate the device and device-side control means that controls the device; and the medical terminal device may be attached to the terminal device attachment means, and thus the device-side control means may stop a function of the device-side operation means.

According to the present invention, the operation information input from the medical terminal device and the operation information input from the device-side operation means are not confused, and the medical care device can be correctly operated based on the operation information input from the medical terminal device. Thus, the medical care can be performed more safely.

### ADVANTAGEOUS EFFECTS OF INVENTION

The present invention provides a medical terminal device that specifies an operator, a patient and a medical care device to be used so that correct medical care can be performed in accordance with the operator and the patient by use of the desired medical care device, and a medical care system including such a medical terminal device.

### BRIEF DESCRIPTION OF DRAWINGS

[FIG. 1] FIG. 1 is a schematic overall view of a medical system.
[FIG. 2] FIG. 2 is a block diagram showing elements of the medical system.
[FIG. 3] FIG. 3 shows patient information stored on a database.
[FIG. 4] FIG. 4 shows operator information stored on a database.
[FIG. 5] FIG. 5 shows medical care device information stored on a database.
[FIG. 6] FIG. 6 is a flowchart showing a medical care process performed by the medical system.
[FIG. 7] FIG. 7 shows a first operation screen of a medical care unit.
[FIG. 8] FIG. 8 shows a second operation screen of the medical care unit.
[FIG. 9] FIG. 9 shows an operation screen of a panorama X-ray imaging device.
[FIG. 10] FIG. 10 shows an operation screen of a root canal length measurement device.
[FIG. 11] FIG. 11 shows an operation screen of an X-ray CT imaging device.
[FIG. 12] FIG. 12 shows an operation screen of a laser treatment device.
[FIG. 13] FIG. 13 is a flowchart showing an operation unit switching process.

### DESCRIPTION OF EMBODIMENTS

Hereinafter, a medical system 1 according to the present invention will be described with reference to the drawings.

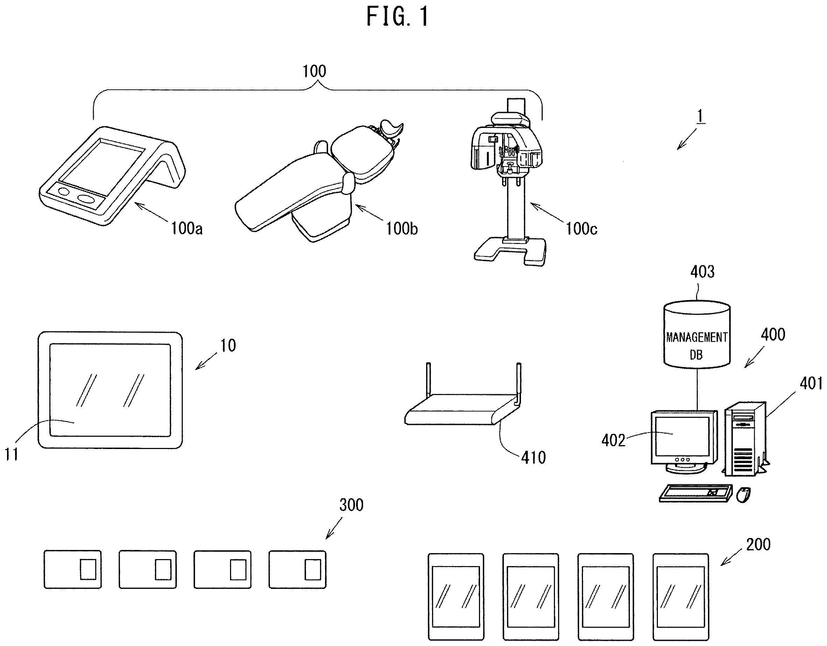

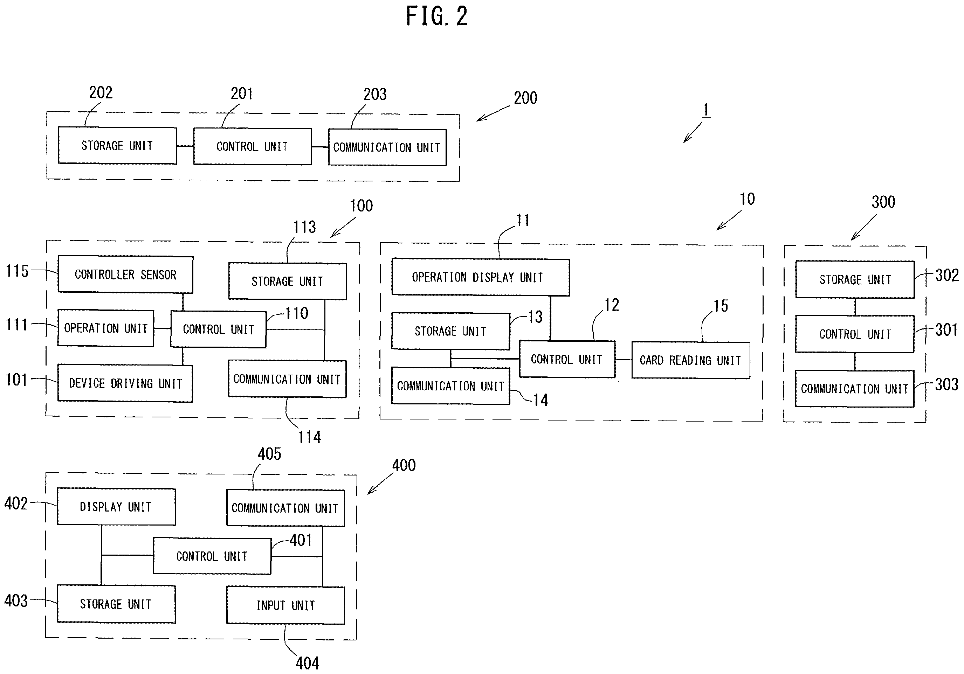

FIG. 1 is a schematic overall view of the medical system 1, and FIG. 2 is a block diagram showing elements of the medical system 1.

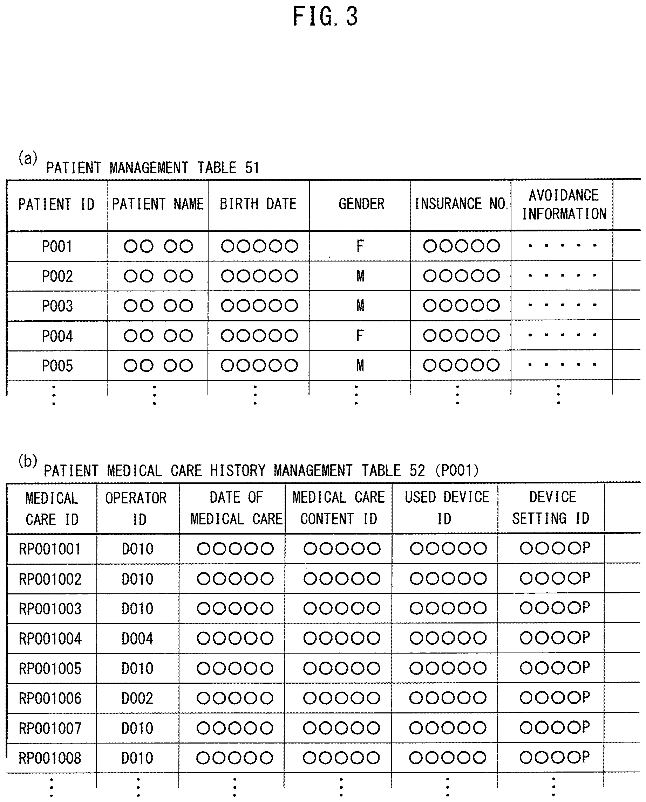

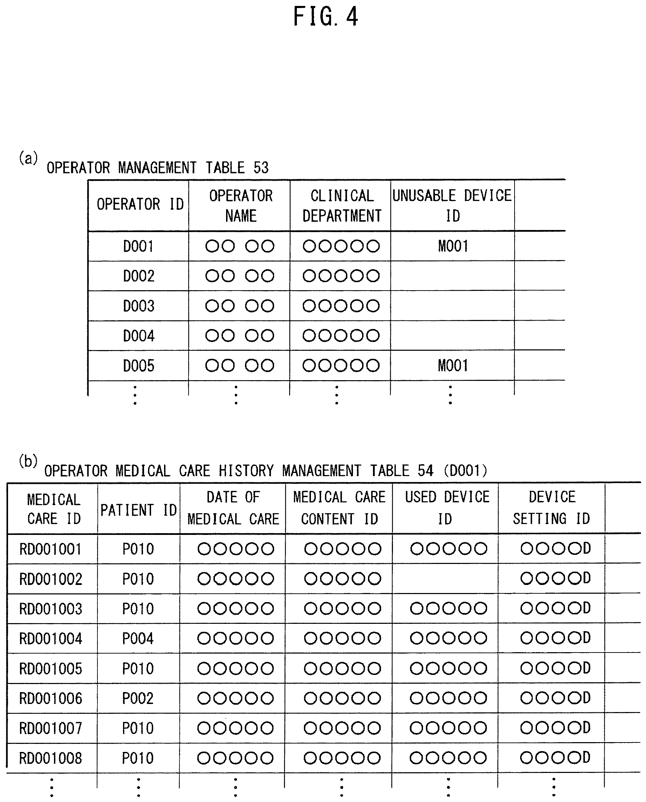

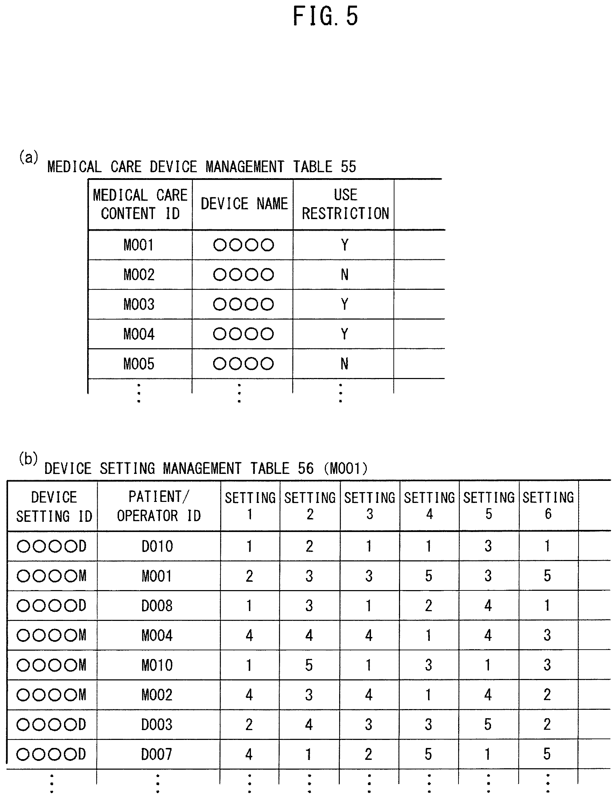

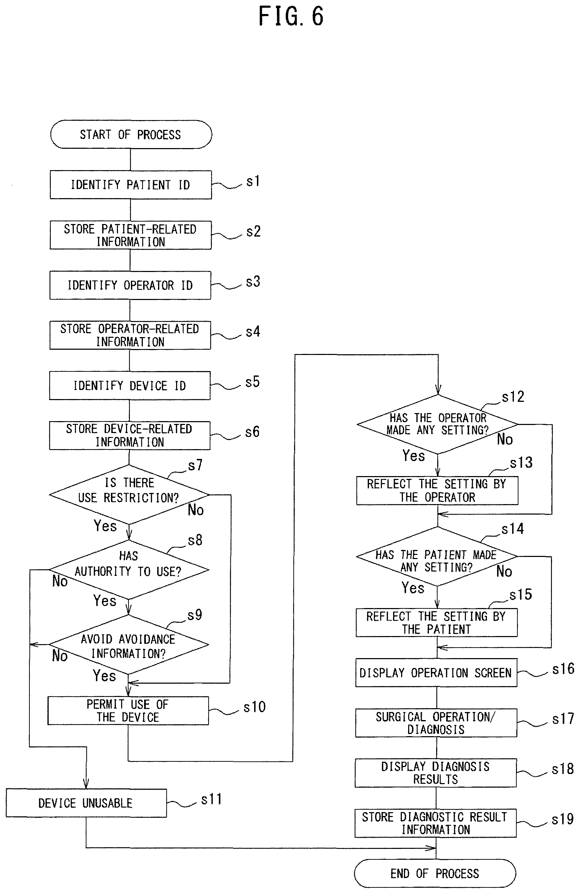

FIG. 3 shows patient information stored on a database. FIG. 4 shows operator information stored on a database. FIG. 5 shows medical care device information stored on a database. FIG. 6 is a flowchart showing a medical care process performed by the medical system 1.

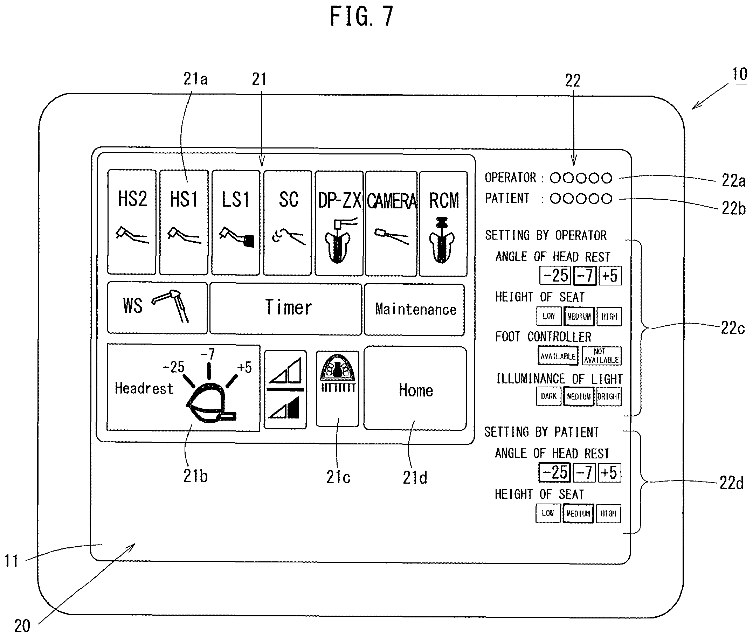

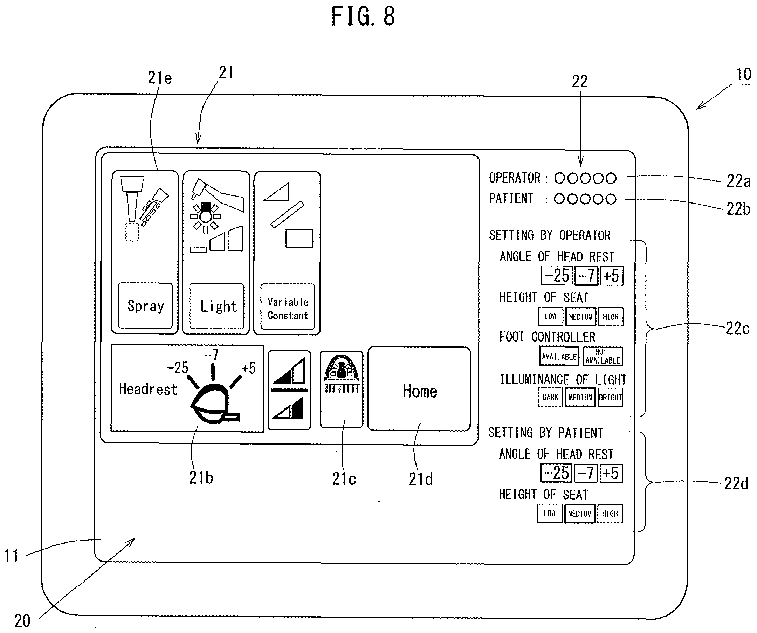

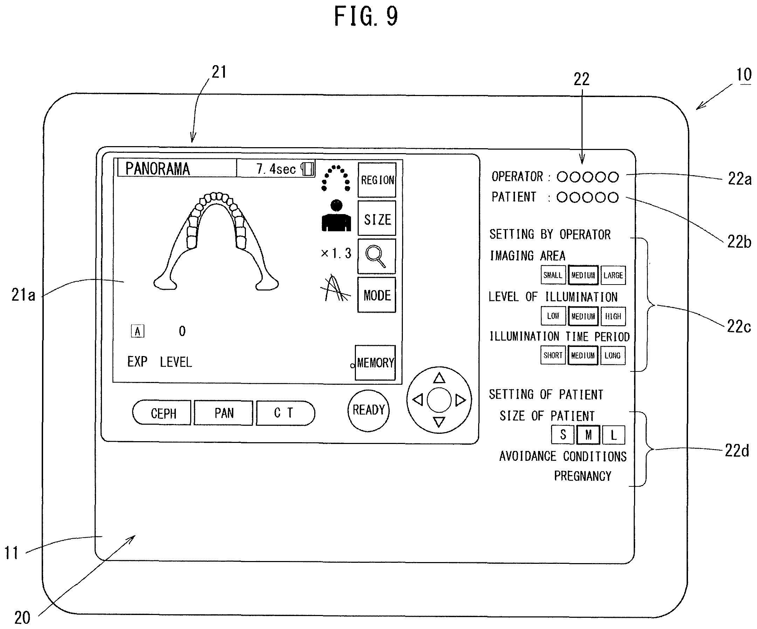

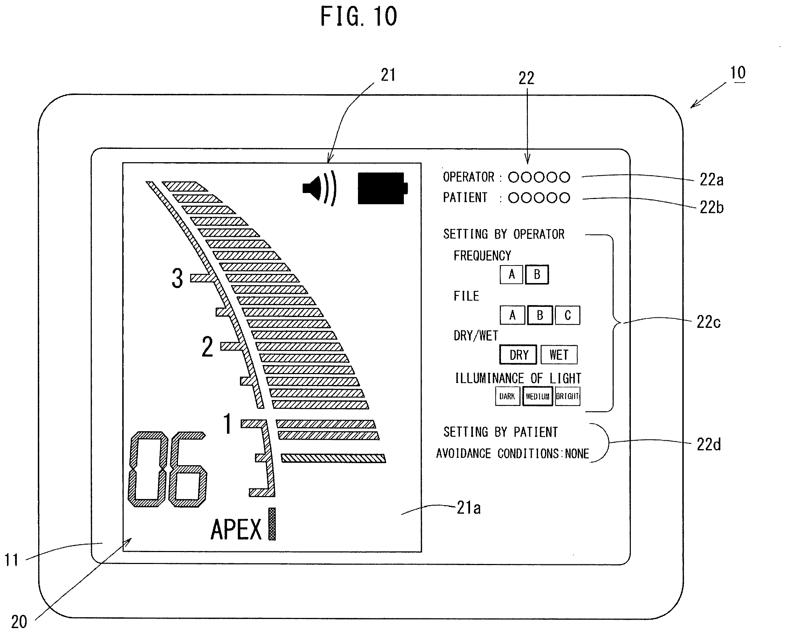

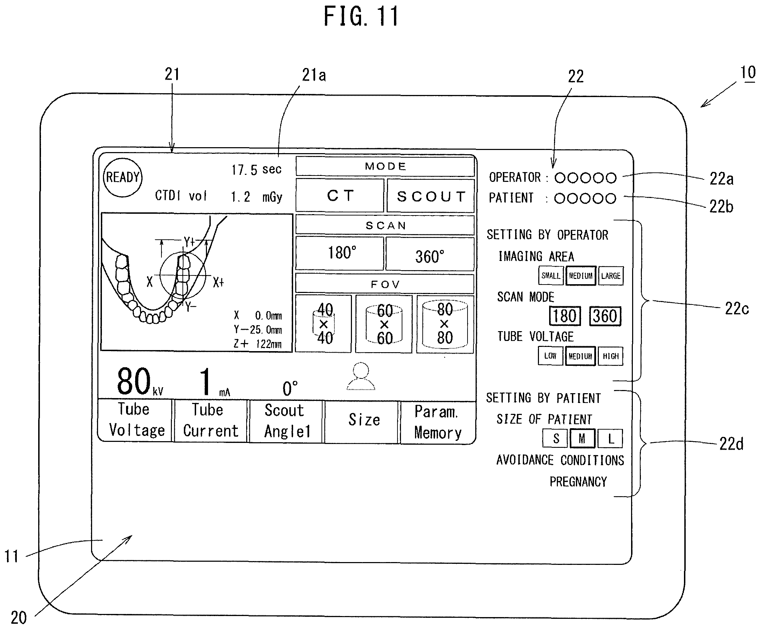

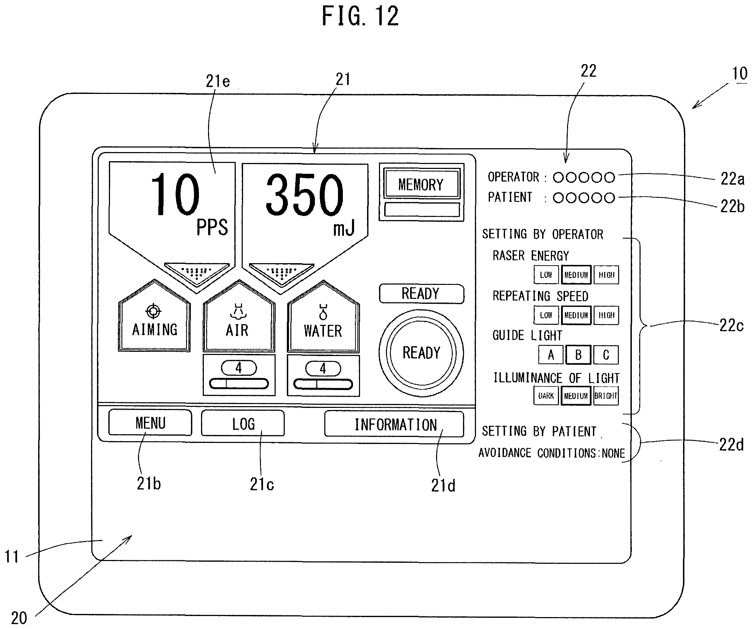

FIG. 7 shows a first operation screen of a medical care unit 100b. FIG. 8 shows a second operation screen of the medical care unit 100b. FIG. 9 shows an operation screen of a panorama X-ray imaging device 100c. FIG. 10 shows an operation screen of a root canal length measurement device 100a. FIG. 11 shows an operation screen of an X-ray CT imaging device. FIG. 12 shows an operation screen of a laser treatment device.

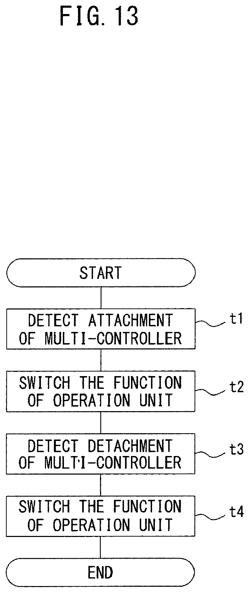

FIG. 13 is a flowchart showing an operation unit switching process.

As shown in FIG. 1, the medical system 1 includes a multi-controller 10, a plurality of types of medical care devices 100, mobile terminals 200 respectively provided for a plurality of patients, identification cards 300 respectively provided for a plurality of operators, a management server 400, and an access point 410 that constructs a wireless LAN environment.

The medical care device 100 is, for example, a peripheral medical care device such as a root canal length measurement device 100a or the like, a medical care unit 100b, a panorama X-ray imaging device 100c, an X-ray CT imaging device, a laser treatment device or the like. The medical care device 100 is not limited to these and may be any of various medical care devices provided in accordance with the content of the medical care.

The multi-controller 10 is a so-called tablet type mobile terminal. As shown in FIG. 2, the multi-controller 10 includes a touch panel operation display unit 11 formed of a liquid crystal display, a control unit 12, a storage unit 13, a communication unit 14, a card read unit 15 and the like.

The operation display unit 11 is located in a front surface part of the multi-controller 10. The operation display unit 11 acts as a display device that displays an operation screen 20 for each of the medical care devices 100 as shown in FIG. 7 through FIG. 12, and also acts as an operation device that allows an operation to be input by a touch or slide operation.

The control unit 12 is formed of a CPU, and executes various types of control processes in accordance with programs stored on the storage unit 13.

The storage unit 13 is formed of a ROM, a RAM, a hard disc or the like, and stores various types of programs including control programs that control various types of devices, self-identification information, log data or the like.

The communication unit 14 is a wireless communication device, such as a LAN board or the like, that is connectable to a wireless network environment via an access point 410.

The card read unit 15 is a device that reads an identifier which is stored on a storage unit 302 of an identification card 300 described later. In the case where the identification card 300 is a contactless authentication card such as an RFID card or the like, the card read unit 15 acts as a reader device. In the case where the identification card 300 is a contact authentication card or a magnetic card, the card read unit 15 acts as a reading device of a format in accordance with the respective type of card.

In the multi-controller 10 having such a structure, the operation display unit 11, the storage unit 13, the communication unit 14 and the card read unit 15 described above are connected to, and controlled by, the control unit 12.

The medical care device 100 such as the root canal length measurement device 100a or the like includes a device driving unit 101 that performs the respective medical care as well as a control unit 110 that controls the device driving unit 101, an operation unit 111 that allows an operator to make an operation input, a storage unit 113, a communication unit 114, a controller sensor 115 and the like.

The control unit 110 is formed of a CPU, and executes various types of control processes in accordance with programs stored on the storage unit 113.

The storage unit 113 is formed of a ROM, a RAM, a hard disc or the like, and stores various types of programs including control programs that control various types of devices, self-identification information, log data or the like.

The communication unit 114 is a wireless communication device such as a LAN board or the like that is connectable to a wireless network environment via the access point 410.

The controller sensor 115 is a sensor that detects attachment of the multi-controller 10 to an attachment portion (not shown) formed in the medical care device 100.

In the medical care device 100 having such a structure, the operation unit 111, the storage unit 113, the communication unit 114 and the controller sensor 115 described above are connected to, and controlled by, the control unit 110.

The mobile terminal 200 is carried by a patient, and includes a control unit 201, a storage unit 202, and a communication unit 203. In the case where the mobile terminal 200 is a smart phone, a PDA or the like, the mobile terminal 200 includes an appropriate device such as an operation display unit or the like.

The control unit 201 is formed of a CPU, and executes various types of control processes in accordance with programs stored on the storage unit 202.

The storage unit 202 is formed of a ROM, a RAM or the like, and stores various types of programs including control programs that control various types of devices, self-identification information, log data or the like.

The communication unit 203 is a wireless communication device such as a LAN board or the like that is connectable to a wireless network environment via an access point 410.

In the mobile terminal 200 having such a structure, the storage unit 202 and the communication unit 203 described above are connected to, and controlled by, the control unit 201.

The identification card 300 includes a control unit 301, a storage unit 302, a communication unit 303 and a receiving coil that receives radio waves from a reader of an electromagnetic induction system. The identification card 300 is a contactless authentication card, such as an RFID card or the like, that is carried by the operator. Alternatively, the identification card 300 may be a contact authentication card or a magnetic card.

The control unit 301 is a device that is formed of a one-chip IC (integrated circuit) and executes various types of control processes in accordance with programs stored on the storage unit 302.

The storage unit 302 is incorporated into the one-chip IC (integrated circuit) and stores various types of programs including control programs that control various types of devices, self-identification information, log data or the like.

The communication unit 303 is a wireless communication device such as a LAN board or the like that is connectable to a wireless network environment via the access point 410.

In the identification card 300 having such a structure, the storage unit 302 and the communication unit 303 described above are connected to, and controlled by, the control unit 301.

The management server is a computer, and includes a control unit 401, a display unit 402, a storage unit 403, an operation device as an input unit 404 such as a mouse, a keyboard or the like, a communication unit 405 communicable with the access point 410, and a storage medium read device that reads information from various types of storage mediums such as a DVD-RAM and the like or a storage medium read/write device.

The control unit 401 is formed of a CPU, a ROM and a RAM, and executes various types of control processes in accordance with programs stored on the storage unit 403.

The display unit 402 is a device that is formed of a liquid crystal monitor, a CRT display or the like and displays various types of information.

The storage unit 403 is formed of a hard disc or the like, and stores various types of data or various types of programs including control programs that control various types of devices as well as a management database (DB) described later.

The management server 400 has a web server function, a database server function and a mail server function.

In more detail, the storage unit 403 of the management server 400 stores and manages, by use of the management database (DB), patient information, medical care history information on patients, operator information, medical care history information on operators, medical care device information, information on a setting made on the medical care devices by the patients and the operators, and information necessary to administer the medical care system 1.

As shown in FIG. 3(a), which shows a patient management table 51, the patient information includes a unique patient ID set for each registered patient as well as information on the patient including the birth date, gender, insurance number, avoidance information indicating that, for example, the patient is pregnant and thus cannot be subjected to X-ray photography, avoidance information listing drugs that cannot be administered to the patient because of allergy, and other information.

As shown in FIG. 3(b), which is a patient medical care history management table 52, the patient medical care history information is managed in the form of a management table provided for each registered patient. The patient medical care history information includes a unique medical care ID set for each medical care, an operator ID of the operator who was in charge of the medical care, date of the medical care, a corresponding medical care content ID among medical care content IDs uniquely set for medical care contents respectively, a used device IC indicating an ID of the used medical care device among medical care device IDs uniquely set for medical care devices respectively, a corresponding device setting ID among device setting IDs uniquely set for settings on the medical care device respectively, and other medical care history information on the patient. Among the numerical figures of each medical care ID, the four numerical figures after "R", namely, the second through fifth numerical figures correspond to the patient ID, so that the patient who received the medical care having the medical care ID is distinguished.

As shown in FIG. 4(a), which is an operator management table 53, the operator information includes a unique operator ID set for each registered operator, the clinical department in which the operator is specialized, an unusable device ID indicating an ID of a medical care device which does not fulfill a use restriction condition set for each medical care device and thus cannot be used, and other information on the operator. Herein, the term "operator" represents a concept encompassing a doctor as well as a person capable of providing medical care, diagnosis, surgical operation, prescription and the like such as a pharmacist, a therapist, a practitioner of acupuncture and moxibustion, an osteopath and the like.

As shown in FIG. 4(b), which is an operator medical care history management table 54, the operator medical care history information is managed in the form of a management table provided for each registered operator. The operator medical care history information includes a unique medical care ID set for each medical care, a patient ID of the patient to whom the operator provided medical care, date of the medical care, a medical care content ID as described above, a used device ID as described above, a device setting ID as described above, and other medical care history information on the operator. Among the numerical figures of each medical care ID, the four numerical figures after "R", namely, the second through fifth numerical figures correspond to the operator ID, so that the operator who provided the medical care having the medical care ID is distinguished. When the medical care is made once, a medical care ID by which the patient can be specified and a medical care ID by which the operator can be specified are provided, so that a management table is made for each patient and for each operator. Alternatively, the patients and the operators may be managed by one management table.

As shown in FIG. 5(a), which is a medical care device management table 55, the medical care device information includes a unique medical care device ID set for each registered medical care device, the name of the device, use restriction indicating whether or not the device fulfills the use restriction condition for the device, and other information on the medical care device.

As shown in FIG. 5 (b) , which is a device setting management table 56, the information on a setting made on the medical care device by the patient or the operator is managed in the form of a management table provided for each registered medical care device . The information on the setting made on the medical care device by the patient or the operator includes a unique device setting ID set for each setting, a patient/operator ID indicating the patient ID or the operator ID of the patient or the operator who made the setting, settings 1 through 6 representing the setting content for each setting item, and other medical care history information on the setting made on the medical care device.

In FIG. 5(b) , settings 1 through 6 are shown in accordance with the number of setting items which can be set on the medical care device. The number of settings is not limited to six, and any number of settings may be provided in accordance with the number of setting items which can be set on the corresponding medical care device.

In the case where, for example, the medical care device 100 is a medical care unit 100b, the following setting items may be set, for example. Setting 1 may be on the angle of the heat rest, setting 2 may be on the illuminance of the light, setting 3 may be on the height of the seat, setting 4 may be on the raising angle of the backseat, setting 5 may be on the speed of driving to raise the backseat, and setting 6 may be on whether the foot controller is available or not.

The medical system 1 including the multi-controller 10, the medical care devices 100, the mobile terminals 200, the identification cards 300 and the management server 400 structured as described above will be described with reference to the flowchart in FIG. 6. In the following, the medical care process performed by use of the medical care unit 100b will be described. The present invention is not limited to this, and substantially the same flow is used unless a process specific to the medical care device is performed.

At the start of the medical care process, the multi-controller 10 identifies the patient ID, which is a patient identifier that is stored on the storage unit 202 of the mobile terminal 200 and transmitted/received via the communication unit 203 and the access point 410 (step s1) . In the case where patient IDs of a plurality of patients are identified at the same time, the patient ID of the patient to receive the medical care may be selectively approved and thus identified.

The multi-controller 10 which has identified the patient ID accesses the management server 400 via the communication unit 14 and the access point 410, retrieves patient-related information on the patient stored in the patient management table 51 or the patient medical care history management table 52 on the storage unit 403 of the management server 400, and temporarily stores such information on the storage unit 13 (step s2).

Next, the multi-controller 10 identifies the operator ID, which is an operator identifier that is stored on the storage unit 302 of the identification card 300 and transmitted/received via the card read unit 15 and the communication unit 303 (step s3) .

The multi-controller 10 which has identified the operator ID accesses the management server 400 via the communication unit 14 and the access point 410, retrieves operator-related information on the operator stored in the operator management table 53 or the operator medical care history management table 54 on the storage unit 403 of the management server 400, and temporarily stores such information on the storage unit 13 (step s4). The order of the identification of the patient ID and the identification of the operator ID may be opposite from the above.

The multi-controller 10 which has identified the patient ID and the operator ID and stored the related information on the storage unit 13 in this manner is attached to an attachment portion (not shown) of the medical care device 100 to be used. When being thus attached, the multi-controller 10 identifies the medical care device 100 to which the multi-controller 10 is attached (step 5) .

The multi-controller 10 is attached to the attachment portion so as to control the operation of the medical care device 100 by wireless communication. Alternatively, the multi-controller 10 may be attached so as to control the operation of the medical care device 100 by wired communication, namely, by electric connection.

The medical care device to be used may be identified as follows. A medical care device ID of a medical care device 100 located closest to the multi-controller 100 is identified. In the case where medical care device IDs of a plurality of medical care devices 100 are identified at the same time, the medical care device ID of the medical care device to be used may be selective approved and thus identified.

The medical care device 100 includes the operation unit 111 and thus the following may be performed. The controller sensor 115 detects that the multi-controller 10 has been attached to the attachment portion. The detection by the controller sensor 115 of the attachment of the multi-controller 10 invalidates input operations on the device driving unit 101 made by the operation unit 111, and switches valid input operations to those made by the multi-controller 10.

This will be described in more detail with reference to FIG. 13. When the controller sensor 115 detects that the multi-controller 10 has been attached to the attachment portion (step t1), the control unit 110 invalidates input operations on the device driving unit 101 made by the operation unit 111, and switches valid input operations to those made by the multi-controller 10 (step t2). By contrast, when the controller sensor 115 detects that the multi-controller 10 has been detached from the attachment portion (step t3), the control unit 110 invalidates input operations on the device driving unit 101 made by the multi-controller 10, and switches valid input operations to those made by the operation unit 111 (step t4).

As shown in FIG. 6, the multi-controller 10 which has identified the medical care device ID accesses the management server 400 via the communication unit 14 and the access point 410, retrieves medical care device-related information on the medical care device stored in the medical care device management table 55 or the device setting management table 56 on the storage unit 403 of the management server 400, and temporarily stores such information on the storage unit 13 (step s6).

The multi-controller 10 which has temporarily stored the medical care device-related information on the storage unit 13 refers to the use restriction information in the medical care device management table 55 to check whether or not the medical care device 100 to be used has a use restriction condition. When the medical care device 100 to be used has a use restriction condition (s7: Yes), the multi-controller 10 checks whether or not the medical care device ID of the medical care device 100 corresponds to an unusable device ID stored in the operator management table 53 among the operator-related information which is regarding the operator identified in step s3 and is temporarily stored on the storage unit 13.

When the medical care 100 device to be used does not correspond to any unusable device for the operator, namely, when the operator has an authority to use the medical care device 100 (step s8: Yes), the multi-controller 10 checks whether or not the medical care device 100 to be used corresponds to the avoidance information stored in the patient management table 51 among the patient-related information which is regarding the patient identified in step s1 and is temporarily stored on the storage unit 13.

When the medical care device 100 to be used does not correspond to the avoidance information on the patient, namely, when the medical care device 100 to be used avoids the avoidance information on the patient (step s9: Yes), the multi-controller 10 permits use of the medical care device 100 in the medical care performed on the patient identified in step s1 by the operator identified in step s3 (step s10).

When the medical care device 100 to be used does not have any use restriction condition in step s7 (s7: No), the multi-controller 10 permits use of the medical care device 100 in the medical care performed on the patient identified in step s1 by the operator identified in step s3 (step s10).

When the medical care device 100 to be used corresponds to the unusable device for the operator, namely, when the operator does not have the authority to use the medical care device 100 (step s8: No) , or when the medical care device 100 to be used corresponds to the avoidance information on the patient (step s9: No), the multi-controller 10 determines that the medical care device 100 is unusable (step s11) and terminates the process.

The multi-controller 10 checks whether or not any of the settings stored in the device setting management table 56 has been made by the operator identified in step s3 on the medical care device 100 permitted to be used. When the operator has made any of the settings (step s12: Yes), the multi-controller 10 reflects the setting on the medical care device 100 (step s13).

Next, the multi-controller 10 checks whether or not any of the settings stored in the device setting management table 56 has been made by the patient identified in step s1 on the medical care device 100 permitted to be used. When the patient has made any of the settings (step s14: Yes), the multi-controller 10 reflects the setting on the medical care device 100 (step s15).

In the case where the patient has made a setting on the setting item on which the operator has also made a setting, the setting made by the patient is prioritized and the setting made by the operator is switched to the setting made by the patient. Alternatively, the setting made by the operator may be prioritized. Still alternatively, a weight may be set for each of the setting made by the patient and the setting made by the operator, so that the setting having a heavier weight is prioritized.

When the operator has made no setting (step s12: No), the multi-controller 10 directly reflects the setting made by the patient on the medical care device in step s15.

The operation display unit 11 of the multi-controller 10 displays an operation screen 20 which reflects the setting made by the operator and the setting made by the patient, or the setting made by the operator or the patient (step s16, see FIG. 7).

As shown in FIG. 7, the operation screen 20 for the medical care unit 100b displayed on the operation display unit 11 includes an upper left operation screen display area 21 and a setting condition display area 22.

The operation screen display area 21 displays, for example, instrument selection icons 21a used to select an instrument, a headrest setting icon 21b used to set the angle of the headrest with respect to the backseat of the medical care unit, an illuminance setting icon 21c used to set the illuminance of the light that illuminates a site on which the surgical operation is to be made, and a Home icon 21d used to jump the screen to a Home screen.

The setting condition display area 22 includes an operator name display 22a that displays the name of the operator, a patient name display 22b that displays the name of the patient, an operator setting display 22c that displays the content of the settings made by the operator, and a patient setting display 22d that displays the content of the settings made by the patient.

In the setting condition display area 22, the setting on the heat rest is displayed in both of the operator setting display 22c and the patient setting display 22d. The setting by the patient to be prioritized is reflected.

On the operation screen 20 displayed on the operation display unit 11 of the multi-controller 10, the operator selects one of the instrument selection icons 21a of the operation screen display area 21. When this occurs, the multi-controller 10 performs the process shown in the above-described flowchart from step s5.

It is now assumed that, for example, the-second-from-the-left turbine is selected among the instrument selection icons 21a. In this case, based on the medical care device ID of the turbine, the multi-controller 10 checks whether or not the turbine has a use restriction condition (step s7), whether or not the operator has an authority to use the turbine (step s8), and the avoidance information on the patient (step s9). When it is determined that the turbine is permitted to be used, the multi-controller 10 reflects the settings by the operator and the patient on the medical care device 100 (steps s13 and s15) and displays the operation screen 20 used to operate the turbine on the operation display unit 11 (step s16, see FIG. 8).

As shown in FIG. 8, the operation screen 20 for the turbine includes the operation screen display area 21 and the setting condition display area 22 like the operation screen 20 for the medical care unit 100b.

The operation screen display area 21 for the turbine displays operation icons 21e used to operate the turbine, the headrest setting icon 21b used to set the angle of the headrest with respect to the backseat of the medical care unit, the illuminance setting icon 21c used to set the illuminance of the light that illuminates a site on which the surgical operation is to be made, the Home icon 21d used to jump the screen to the Home screen, and the like.

The setting condition display area 22 for the turbine includes the operator name display 22a that displays the name of the operator, the patient name display 22b that displays the name of the patient, the operator setting display 22c that displays the content of the settings made by the operator regarding the turbine, and the patient setting display 22d that displays the content of the settings made by the patient regarding the turbine.

The operator operates the operation icons 21e on the operation screen 20 for the turbine to perform a surgical operation and provide diagnosis (step s17). The operator explains the results to the patient while having the results displayed on the operation display area 11 (step s18), and stores the results in the patient medical care history management table 52 and the operator medical care history management table 53 as result information (step s19). Thus, the medical care process is completed.

In the case where the panorama X-ray imaging device 100c is used instead of the medical care unit 100b, the operation display unit 11 displays the operation screen 20 for the panorama X-ray imaging device 100c (see FIG. 9).

The operation screen 20 for the panorama X-ray imaging device 100c includes the operation screen display area 21 and the setting condition display area 22 like the operation screen 20 for the medical care unit 100b. The operation screen 20 is used to make operations, and thus panorama imaging performed by the panorama X-ray imaging device 100c is completed and diagnosis is made (step s17) . Then, the operation display unit 11 of the multi-controller 10 displays a panorama image captured in step s17 (step s18, the panorama image is not shown) , so that the operator can explain the diagnostic results to the patient while showing the panorama image displayed on the operation display unit 11 to the patient.

As described above, the medical system 1 includes the multi-controller 10 which includes the operation display unit 11 that displays the operation screen 20 used to operate the medical care device and also includes the control unit 12 that identifies the operator ID of the operator who is to perform the medical care, the patient ID of the patient who is to receive the medical care and the medical care device ID of the medical care device 100 to be used for the medical care; a plurality of medical care devices 100; and the storage unit 403. The storage unit 403 stores medical care device information stored in the medical care device management table 55 and the device setting management table 56 regarding a plurality of medical care devices 100, operator information stored in the operator management table 53 and the operator medical care history management table 54 regarding a plurality of operators, and patient information stored in the patient management table 51 and the patient medical care history management table 52 regarding a plurality of patients. Based on the operator information corresponding to the operator identified in step s3, the patient information corresponding to the patient identified in step s1 and the medical care device information corresponding to the medical care device 100 identified in step s5, the control unit 12 displays, on the operation display unit 11, the operation screen 20 regarding the medical care device 100 to be used for the medical care as the operation screen 20 which reflects the settings made by the operator to perform the medical care and the settings made by the patient to receive the medical care. Therefore, the operator to perform the medical care is identified among the plurality of operators, the patient to receive the medical care is identified among the plurality of patients, and the medical care device 100 to be used is identified among the plurality of medical care devices 100. Thus, medical care in accordance with the operator and the patient can be correctly performed by use of the desired medical care device 100.

This will be described in more detail. The multi-controller 10 includes the operation display unit 11 that displays the operation screen 20 used to operate the medical care device 100, and the control unit 12 that identifies the operator ID of the operator who is to perform the medical care, the patient ID of the patient who is to receive the medical care, and the medical care device ID of the medical care device 100 to be used for the medical care. Therefore, the operator, the patient and the medical care device 100 can be correctly identified among the plurality of operators, the plurality of patients and the plurality of medical care devices 100.

The medical system 1 further includes the plurality of medical care devices 100; and the storage unit 403 that stores medical care device information stored in the medical care device management table 55 and the device setting management table 56 regarding the plurality of medical care devices 100, operator information stored in the operator management table 53 and the operator medical care history management table 54 regarding the plurality of operators, and patient information stored in the patient management table 51 and the patient medical care history management table 52 regarding the plurality of patients. Based on the operator information corresponding to the operator identified in step s3, the patient information corresponding to the patient identified in step s1 and the medical care device information corresponding to the medical care device 100 identified in step s5, the control unit 12 displays, on the operation display unit 11, the operation screen 20 regarding the medical care device 100 to be used for the medical care as the operation screen which reflects the settings made by the operator to perform the medical care and the settings made by the patient to receive the medical care. Therefore, medical care in accordance with the identified operator and the identified patient can be correctly performed by use of the desired medical care device 100.

The operator ID is stored on the storage unit 302 of the identification card 300, the patient ID is stored on the storage unit 202 of the mobile terminal 200, and the medical care device ID is stored on the storage unit 113 of the medical care device 100. In addition, the multi-controller 10 communicates with the storage units 113, 202 and 302 to identify the operator, the patient and the medical care device 100. Therefore, the operator, the patient and the medical care device can be identified more correctly, and appropriate medical care can be performed.

This will be described in more detail. The medical system 1 includes the storage unit 302 that stores the operator ID, the storage unit 202 that stores the patient ID, and the storage unit 113 that stores the medical care device ID. Therefore, the operator, the patient and the medical care device 100 are identified based on inherent identification information stored on the storage units 113, 202 and 302. Thus, the identification can be performed more correctly.

The operator information includes the unusable device ID which is considered as use permission information regarding the device which the operator is permitted to use. Based on the unusable device ID for the identified operator, the control unit 110 provides a switch between permission and restriction on the use of the medical care device 100 identified in step s5.

Therefore, based on the unusable device ID, the medical care device 100 which needs to be used after being permitted to be used is prevented from being used for the medical care by an operator who is not authorized to use the medical care device 100.

The medical care device 100 includes the communication unit 114, and the multi-controller 10 includes the communication unit 14. The operation information input to the multi-controller 10 is transmitted/received via the communication unit 14, so that the medical care device 100 is controlled by the multi-controller 10. Therefore, the medical care device 100 can be operated by wireless connection without physically connecting the medical care device 100 and the multi-controller 10 to each other. This improves the convenience.

The operation display unit 11 of the multi-controller 10 displays a panorama image as the patient information. Therefore, the multi-controller 10 can be used to display medical care information which is used to explain the medical care results for informed consent, in addition to being used to operate the medical care device 100 to be used. This also improves the convenience. The image displayed as the patient information may be a panorama image, a CT image, an image of a tooth, a fluorescent image, an image of the patient captured by a camera, an intraoral image or the like.

The plurality of medical care devices 100 each include an attachment portion to which the multi-controller 10 is attached. Therefore, the multi-controller 10 capable of operating different types of medical care devices 100 can be attached to the attachment portion of any of these medical care devices. Thus, correct medical care can be performed without using a wrong medical care device.

The attachment of the multi-controller 10 to the attachment portion stops the function of the operation unit 111. Therefore, the operation information input from the multi-controller 10 and the operation information input from the operation unit 111 are not confused, and the medical care device 100 can be correctly operated based on the operation information input from the multi-controller 10. Thus, the medical care can be performed more safely.

In the above description, the operation screen 20 for the medical care unit 100b and the operation screen 20 for the panorama X-ray imaging device 100c are described. In the case where the root canal length measurement device 100a, the X-ray CT imaging device and the laser treatment device are used, the operation screens 20 are respectively as shown in FIG. 10 through FIG. 12.

The operation screen 20 for the root canal measurement device 100a shown in FIG. 10 includes the operation screen display area 21 and the setting condition display area 22. The operation screen display area 21 for the root canal measurement device 100a displays a measurement result display 21a that displays measurement results obtained by the root canal measurement device 100a.

The setting condition display area 22 for the root canal measurement device 100a includes the operator name display 22a that displays the name of the operator, the patient name display 22b that displays the name of the patient, the operator setting display 22c that displays the content of the settings made by the operator regarding the root canal measurement device 100a, and the patient setting display 22d that displays the avoidance information on the patient regarding the root canal measurement device 100a.

The operator performs measurement and provides diagnosis while checking the measurement result display 21a on the operation screen 20 for the root canal measurement device 100a.

The operation screen 20 for the X-ray CT imaging device shown in FIG. 11 includes the operation screen display area 21 and the setting condition display area 22. The operation screen display area 21 displays an imaging condition setting area 21a used to operate the X-ray CT imaging device or the like.

The setting condition display area 22 for the X-ray CT imaging device includes the operator name display 22a that displays the name of the operator, the patient name display 22b that displays the name of the patient, the operator setting display 22c that displays the content of the settings made by the operator regarding the X-ray CT imaging device, and the patient setting display 22d that displays the content of the setting made by the patient regarding the X-ray CT imaging device and the avoidance information.

The operator can set the imaging condition by use of the imaging condition setting area 21a on the operation screen 20 for the X-ray CT imaging device and perform imaging. The operator can explain the imaging results to the patient while having the results displayed on the operation display area 11. The display on the operation screen 20 may be changed so as to set the positioning for CT imaging, using the panorama image captured by the panorama X-ray imaging device 100c as a scout image.

The operation screen 20 for the laser treatment device shown in FIG. 12 includes the operation screen display area 21 and the setting condition display area 22. The operation screen display area 21 displays operation icons 21e used to operate the laser treatment device, a MENU icon 21b, a LOG icon 21c, an INFORMATION icon 21d and the like.

The setting condition display area 22 for the laser treatment device includes the operator name display 22a that displays the name of the operator, the patient name display 22b that displays the name of the patient, the operator setting display 22c that displays the content of the settings made by the operator regarding the laser treatment device, and the patient setting display 22d that displays the avoidance information regarding the laser treatment device.

The operator operates the operation icons 21e on the operation screen 20 for the laser treatment device to perform a surgical operation and provide diagnosis.

In the case where a medical care device other than the medical care devices described above is used, the operation screen 20 includes the operation screen display area 21 and the setting condition display area 22.

The medical care device according to the present invention corresponds to the medical care device 100, the root canal length measurement device 100a, the medical care unit 100b or the panorama X-ray imaging device 100c in the above-described embodiment; and similarly,
the operator identification means that identifies the operator corresponds to the control unit 12 that identifies the operator ID;
the patient identification means that identifies the patient corresponds to the control unit 12 that identifies the patient ID;
the device identification means that identifies the medical care device corresponds to the control unit 12 that identifies the medical care device ID;
the medical terminal device corresponds to the multi-controller 10;
the operator information on the operator corresponds to the information stored in the operator management table 53 and the operator medical care history management table 54;
the patient information on the patient corresponds to the information stored in the patient management table 51 and the patient medical care history management table 52;
the information storage means corresponds to the storage unit 403;
the control means corresponds to the control unit 401;
the identification information inherent to the operator corresponds to the operator ID;
the operator identification information storage means corresponds to the storage unit 302;
the identification information inherent to the patient corresponds to the patient ID;
the patient identification information storage means corresponds to the storage unit 202;
the identification information inherent to the medical care device corresponds to the medical care device ID;
the device identification information storage means corresponds to the storage unit 113;
the terminal device attachment means corresponds to the attachment portion;
the device-side operation means corresponds to the operation unit 111;
the device-side control means corresponds to the control unit 110;
the use permission information corresponds to the unusable device ID;
the use restriction means corresponds to the control unit 12 that permits use of the device in step s10;
the wireless communication means corresponds to the communication unit 14; and
the patient medical care information corresponds to the X-ray image.

However, the present invention is not limited to the structure of the above-described embodiment, and may be carried out in any of various embodiments.

For example, in the above description, the identification card 300 is used to identify the operator. Alternatively, the identification card 300 may be used as a patient ID card so that the patient is identified by the identification card 300 instead of the mobile terminal 200. By contrast, the operator may use the mobile terminal 200 which includes a PHS-type telephone device instead of the identification card 300.

In the above description, the operator information is used to set the authority to use the medical care device 100 and put the device into an unusable state. Alternatively, a combination of the operator information and the medical care device information is used as a safety switch of the medical care device 100. In this case, only when these two pieces of information are stored and set, the medical care device 100 may be turned on and made usable.

In the above description, the operation screen 20 regarding the medical care device 100 to be used for the medical care is displayed on the operation display unit 11 as an operation screen which reflects the settings made by the operator who is to perform the medical care and settings made by the patient who is to receive the medical care. In addition, the settings on the medical care device 100 also reflect the settings made by the operator who is to perform the medical care and settings made by the patient who is to receive the medical care. Alternatively, the settings made by the operator who is to perform the medical care and settings made by the patient who is to receive the medical care may be reflected only on the operation screen, and these settings may be reflected on the settings on the medical care device 100 after being approved on the operation screen.

The patient information, the operator information and the like may be stored on the storage unit 13 of the multi-controller 10 instead of the storage unit 403 of the management server 400. In this case, the management server 400 may be omitted.

In the above description, based on the patient information, the operator information and the medical care device information, the control unit 12 of the multi-controller 10 displays, on the operation display unit 11, the operation screen 20 regarding the medical care device 10 to be used for the medical care as an operation screen which reflects the settings made by the operator who is to perform the medical care and settings made by the patient who is to receive the medical care. Alternatively, the control unit 401 of the management server 400 may cause the settings made by the operator who is to perform the medical care and settings made by the patient who is to receive the medical care to be reflected on the operation screen regarding the medical care device 100 used for the medical care, and transmit the information on the operation screen which reflects the settings to the multi-controller 10, so that the multi-controller 10 displays the information on the operation display unit 11.

The operation screen 20 displayed on the operation display unit 11 of the multi-controller 10 may display a maintenance personnel icon used to perform a maintenance operation. In the case where information on the maintenance personnel is stored as the operator ID, it preferably improves the convenience to display an operation display screen for the maintenance personnel on the operation display unit 11.

### REFERENCE SIGNS LIST

- 1 ...: Medical system
- 10 ...: Multi-controller
- 11 ...: Operation display unit
- 12, 110, 401 ...: Control unit
- 14 ...: Communication unit
- 20 ...: Operation screen
- 51 ...: Patient management table
- 52 ...: Patient medical care history management table
- 53 ...: Operator management table
- 54 ...: Operator medical care history management table
- 55 ...: Medical care device management table
- 56 ...: Device setting management table
- 100 ...: Medical care device
- 100a ...: Root canal length measurement device
- 100b ...: Medical care unit
- 100c ...: Panorama X-ray imaging device
- 111 ...: Operation unit
- 113, 202, 302, 403 ...: Storage unit

## Claims

1. A medical system, comprising:
a medical terminal device including
an operation display unit adapted to display an operation screen used to operate a medical care device,
operator identification means adapted to identify an operator to perform medical care,
patient identification means adapted to identify a patient to receive the medical care, and
device identification means adapted to identify a medical care device to be used for the medical care;
a plurality of medical care devices;
information storage means adapted to store device information on the plurality of medical care devices, operator information on a plurality of operators, and patient information on a plurality of patients; and
control means adapted to, based on the operator information corresponding to the operator identified by the operator identification means, the patient information corresponding to the patient identified by the patient identification means, and the device information corresponding to the medical care device identified by the device identification means, display an operation screen on the medical care device to be used for the medical care as an operation screen which reflects a setting made by the operator to perform the medical care and a setting made by the patient to receive the medical care, the operation screen being displayed on the operation display unit,
**characterized in that** the plurality of medical care devices each include terminal device attachment means to which the medical terminal device is attached, and a controller sensor adapted to detect attachment of the medical terminal device,
the medical care device includes device-side operation means used to operate the device and device-side control means adapted to control the device; and
when the medical terminal device is attached to the terminal device attachment means and the controller sensor detects the attachment of the medical terminal device, the device-side control means is adapted to stop a function of the device-side operation means.

2. A medical system according to claim 1, further comprising:
operator identification information storage means that stores identification information inherent to the operator;
patient identification information storage means that stores identification information inherent to the patient; and
device identification information storage means that stores identification information inherent to the medical care device;
wherein the operator identification means, the patient identification means and the device identification means perform identification by respectively communicating with the operator identification information storage means, the patient identification information storage means and device identification information storage means.

3. A medical system according to claim 1 or 2, wherein:
the operator information includes use permission information on the device which the operator is permitted to use;
the medical care device includes use restriction means that provides a switch between permission and restriction on use of the device based on use permission information which permits the device to be used; and
the use restriction means provides a switch between permission and restriction on use of the medical care device identified by the device identification means based on use permission information on the operator identified by the operator identification means.

4. A medical system according to any one of claims 1 through 3, wherein:
the medical care device and the medical terminal device each include wireless communication means; and
operation information input to the medical terminal device is transmitted/received via the wireless communication means, so that the medical care device is operated by the medical terminal device.

5. A medical system according to any one of claims 1 through 4, wherein:
the patient information includes medical care information on the patient; and
the operation display unit displays the medical care information on the patient identified by the patient identification information.

6. A medical terminal device, comprising:
an operation display unit adapted to display an operation screen used to operate a medical care device;
operator identification means adapted to identify an operator to perform medical care;
patient identification means adapted to identify a patient to receive the medical care;
device identification means adapted to identify a medical care device to be used for the medical care; and
communication means adapted to
communicate with information storage means that stores device information on a plurality of medical care devices, operator information on a plurality of operators and patient information on a plurality of patients, and,
based on the operator information corresponding to the operator identified by the operator identification means, the patient information corresponding to the patient identified by the patient identification means and device information corresponding to the medical care device identified by the device identification means, to display an operation screen on the medical device to be used for the medical care as an operation screen which reflects a setting made by the operator to perform the medical care and a setting made by the patient to receive the medical care, the operation screen being displayed on the operation display unit,
**characterized in that** the medical terminal device is attachable to the medical care device so as to control the operation of the medical care device, wherein a device-side control means of the medical care device is adapted to switch a function of a device-side operation means of the medical care device upon attachment or detachment of the medical terminal device, and wherein the device-side control means is adapted to stop a function of the device-side operation means, when the medical terminal device is attached to the terminal device attachment means and a controller sensor of the medical care device detects the attachment of the medical terminal device.

7. A medical terminal device according to claim 6, wherein the operator identification means, the patient identification means and the device identification means perform identification by respectively communicating with the operator identification information storage means that stores identification information inherent to the operator, the patient identification information storage means that stores identification information inherent to the patient, and device identification information storage means that stores identification information inherent to the medical care device.

8. A medical terminal device according to claim 6 or 7, further comprising wireless communication means that wirelessly communicates with device-side wireless communication means included in the medical care device;
wherein input operation information is transmitted from the wireless communication means to the device-side wireless communication means, so that the medical terminal device operates the medical care device.

9. A medical terminal device according to any one of claims 6 through 8, wherein:
the patient information includes medical care information on the patient; and
the operation display unit displays the medical care information on the patient identified by the patient identification means.

## Patentansprüche

1. Medizinisches System, aufweisend:
ein medizinisches Endgerät, welches umfasst
eine Operationsanzeigeeinheit, die dafür geeignet ist, einen Operationsschirm anzuzeigen, der genutzt wird, um eine medizinische Versorgungsvorrichtung zu betreiben,
ein Mittel zur Bediener-Identifizierung, das dafür geeignet ist, einen Bediener, um eine medizinische Versorgung durchzuführen, zu identifizieren,
ein Mittel zur Patienten-Identifizierung, das dafür geeignet ist, einen Patienten, um die medizinische Versorgung zu empfangen, zu identifizieren, und
ein Mittel zur Vorrichtungs-Identifizierung, das dafür geeignet ist, eine medizinische Versorgungsvorrichtung, die für die medizinische Versorgung verwendet werden soll, zu identifizieren;
eine Vielzahl medizinischer Versorgungsvorrichtungen;
ein Mittel zur Informationsspeicherung, das dafür geeignet ist, eine Vorrichtungs-Information über die Vielzahl medizinischer Versorgungsvorrichtungen, eine Bediener-Information über eine Vielzahl von Bedienern und eine Patienten-Information über eine Vielzahl von Patienten zu speichern; und
ein Steuerungsmittel, das dafür geeignet ist, basierend auf der Bediener-Information, die dem durch das Mittel zur Bediener-Identifizierung identifizierten Bediener entspricht, der Patienten-Information, die dem durch das Mittel zur Patienten-Identifizierung identifizierten Patienten entspricht, und der Vorrichtungs-Information, die der durch das Mittel zur Vorrichtungs-Identifizierung identifizierten medizinischen Versorgungsvorrichtung entspricht, einen Operationsschirm auf der medizinischen Versorgungsvorrichtung, die für die medizinische Versorgung verwendet werden soll, als einen Operationsschirm anzuzeigen, der eine Einstellung widerspiegelt, die vom Bediener vorgenommen wird, um die medizinische Versorgung durchzuführen, und eine Einstellung, die vom Patienten vorgenommen wird, um die medizinische Versorgung zu empfangen, wobei der Operationsschirm auf der Operationsanzeigeeinheit angezeigt wird,
**dadurch gekennzeichnet, dass** die Vielzahl medizinischer Versorgungsvorrichtungen jeweils ein Mittel zur Anbringung eines Endgeräts enthält, an dem das medizinische Endgerät angebracht wird, und einen Controller-Sensor, der dafür geeignet ist, eine Anbringung des medizinischen Endgeräts zu detektieren,
die medizinische Versorgungsvorrichtung ein vorrichtungsseitiges Operationsmittel, das genutzt wird, um die Vorrichtung zu betreiben, und ein vorrichtungsseitiges Steuerungsmittel umfasst, das dafür geeignet ist, die Vorrichtung zu steuern; und
wenn das medizinische Endgerät am Mittel zur Anbringung eines Endgeräts angebracht wird und der Controller-Sensor die Anbringung des medizinischen Endgeräts detektiert, das vorrichtungsseitige Steuerungsmittel dafür geeignet ist, eine Funktion des vorrichtungsseitigen Operationsmittels zu stoppen.

2. Medizinisches System nach Anspruch 1, ferner aufweisend:
ein Speichermittel für Informationen einer Bediener-Identifizierung, das eine dem Bediener eigene Identifizierungsinformation speichert;
ein Speichermittel für Informationen einer Patienten-Identifizierung, das eine dem Patienten eigene Identifizierungsinformation speichert; und
ein Speichermittel für Informationen einer Vorrichtungs-Identifizierung, das eine der medizinischen Versorgungsvorrichtung eigene Identifizierungsinformation speichert;
wobei das Mittel zur Bediener-Identifizierung, das Mittel zur Patienten-Identifizierung und das Mittel zur Vorrichtungs-Identifizierung eine Identifizierung durchführen, indem mit dem Speichermittel für Informationen einer Bediener-Identifizierung, dem Speichermittel für Informationen einer Patienten-Identifizierung bzw. dem Speichermittel für Informationen einer Vorrichtungs-Identifizierung kommuniziert wird.

3. Medizinisches System nach Anspruch 1 oder 2, wobei:
die Bediener-Information eine Information über eine Nutzungserlaubnis über die Vorrichtung enthält, die zu nutzen dem Bediener erlaubt ist;
das medizinische Endgerät ein Mittel zur Nutzungsbeschränkung enthält, das eine Umschaltung zwischen Nutzungserlaubnis und -beschränkung der Vorrichtung basierend auf einer Information über eine Nutzungserlaubnis vorsieht, die erlaubt, dass die Vorrichtung genutzt wird; und
das Mittel zur Nutzungsbeschränkung eine Umschaltung zwischen Nutzungserlaubnis und -beschränkung der medizinischen Versorgungsvorrichtung, die durch das Mittel zur Vorrichtungs-Identifizierung identifiziert wird, basierend auf der Nutzungserlaubnisinformation über den Bediener, der durch das Mittel zur Bediener-Identifizierung identifiziert wird, vorsieht.

4. Medizinisches System nach einem der Ansprüche 1 bis 3, wobei:
die medizinische Versorgungsvorrichtung und das medizinische Endgerät jeweils Mittel zur drahtlosen Kommunikation enthalten; und
eine in das medizinische Endgerät eingegebene Operationsinformation über die Mittel zur drahtlosen Kommunikation übertragen/empfangen werden, so dass die medizinische Versorgungsvorrichtung mittels des medizinischen Endgeräts betrieben wird.

5. Medizinisches System nach einem der Ansprüche 1 bis 4, wobei:
die Patienten-Information eine Information einer medizinischen Versorgung über den Patienten enthält; und
die Operationsanzeigeeinheit die Information einer medizinischen Versorgung über den Patienten, der durch das Mittel zur Patienten-Identifizierung identifiziert wurde, anzeigt.

6. Medizinisches Endgerät, aufweisend:
eine Operationsanzeigeeinheit, die dafür geeignet ist, einen Operationsschirm anzuzeigen, der genutzt wird, um eine medizinische Versorgungsvorrichtung zu betreiben,
ein Mittel zur Bediener-Identifizierung, das dafür geeignet ist, einen Bediener, um eine medizinische Versorgung durchzuführen, zu identifizieren,
ein Mittel zur Patienten-Identifizierung, das dafür geeignet ist, einen Patienten, um die medizinische Versorgung zu empfangen, zu identifizieren, und
ein Mittel zur Vorrichtungs-Identifizierung, das dafür geeignet ist, eine medizinische Versorgungsvorrichtung, die für die medizinische Versorgung verwendet werden soll, zu identifizieren; und
Kommunikationsmittel, die dafür geeignet sind,
mit einem Mittel zur Informationsspeicherung zu kommunizieren, das eine Vorrichtungs-Information über eine Vielzahl medizinischer Versorgungsvorrichtungen, eine Bediener-Information über eine Vielzahl von Bedienern und eine Patienten-Information über eine Vielzahl von Patienten speichert, und
basierend auf der Bediener-Information, die dem durch das Mittel zur Bediener-Identifizierung identifizierten Bediener entspricht, der Patienten-Information, die dem durch das Mittel zur Patienten-Identifizierung identifizierten Patienten entspricht, und der Vorrichtungs-Information, die der durch das Mittel zur Vorrichtungs-Identifizierung identifizierten medizinischen Versorgungsvorrichtung entspricht, einen Operationsschirm auf der medizinischen Vorrichtung, die für die medizinische Versorgung verwendet werden soll, als einen Operationsschirm anzuzeigen, der eine Einstellung widerspiegelt, die vom Bediener vorgenommen wird, um die medizinische Versorgung durchzuführen, und eine Einstellung, die vom Patienten vorgenommen wird, um die medizinische Versorgung zu empfangen, wobei der Operationsschirm auf der Operationsanzeigeeinheit angezeigt wird,
**dadurch gekennzeichnet, dass** das medizinische Endgerät an die medizinische Versorgungsvorrichtung anbringbar ist, um eine Operation der medizinischen Versorgungsvorrichtung zu steuern, wobei ein vorrichtungsseitiges Steuerungsmittel der medizinischen Versorgungsvorrichtung dafür geeignet ist, bei Anbringung oder Trennung des medizinischen Endgeräts eine Funktion eines vorrichtungsseitigen Operationsmittels umzuschalten, und wobei das vorrichtungsseitige Steuerungsmittel dafür geeignet ist, eine Funktion des vorrichtungsseitigen Operationsmittels zu stoppen, wenn das medizinische Endgerät am Mittel zur Anbringung eines Endgeräts angebracht wird und ein Controller-Sensor der medizinischen Versorgungsvorrichtung die Anbringung des medizinischen Endgeräts detektiert.

7. Medizinisches Endgerät nach Anspruch 6, wobei das Mittel zur Bediener-Identifizierung, das Mittel zur Patienten-Identifizierung und das Mittel zur Vorrichtungs-Identifizierung eine Identifizierung durchführen, indem mit dem Speichermittel für Informationen einer Bediener-Identifizierung, das eine dem Bediener eigene Identifizierungsinformation speichert, dem Speichermittel für Informationen einer Patienten-Identifizierung, das eine dem Patienten eigene Identifizierungsinformation speichert, bzw. dem Speichermittel für Informationen einer Vorrichtungs-Identifizierung, das eine der medizinischen Versorgungsvorrichtung eigene Identifizierungsinformation speichert, kommuniziert wird.

8. Medizinisches Endgerät nach Anspruch 6 oder 7, ferner aufweisend ein Mittel zur drahtlosen Kommunikation, das mit dem in der medizinischen Versorgungsvorrichtung enthaltenen vorrichtungsseitigen Mittel zur Kommunikation drahtlos kommuniziert;
wobei eine eingegebene Operationsinformation vom Mittel zur drahtlosen Kommunikation zum vorrichtungsseitigen Mittel zur drahtlosen Kommunikation übertragen wird, so dass das medizinische Endgerät die medizinische Versorgungsvorrichtung betreibt.

9. Medizinisches Endgerät nach einem der Ansprüche 6 bis 8, wobei:
die Patienten-Information eine Information einer medizinischen Versorgung über den Patienten enthält; und
die Operationsanzeigeeinheit die Information einer medizinischen Versorgung über den durch das Mittel zur Patienten-Identifizierung identifizierten Patienten anzeigt.

## Revendications

1. Système médical comprenant :
un dispositif de terminal médical comportant
une unité d'affichage de fonctionnement adaptée pour afficher un écran de fonctionnement utilisé pour faire fonctionner un dispositif de soins médicaux,
un moyen d'identification d'opérateur adapté pour identifier un opérateur devant effectuer des soins médicaux,
un moyen d'identification de patient adapté pour identifier un patient devant recevoir les soins médicaux, et
un moyen d'identification de dispositif adapté pour identifier un dispositif de soins médicaux devant être utilisé pour les soins médicaux ;
une pluralité de dispositifs de soins médicaux ;
un moyen de stockage d'informations adapté pour stocker des informations de dispositifs concernant la pluralité de dispositifs de soins médicaux, des informations d'opérateurs concernant une pluralité d'opérateurs, et des informations de patients concernant une pluralité de patients ; et
un moyen de commande adapté pour, sur la base des informations d'opérateur correspondant à l'opérateur identifié par le moyen d'identification d'opérateur, des informations de patient correspondant au patient identifié par le moyen d'identification de patient, et des informations de dispositif correspondant au dispositif de soins médicaux identifié par le moyen d'identification de dispositif, afficher un écran de fonctionnement sur le dispositif de soins médicaux devant être utilisé pour les soins médicaux en tant qu'écran de fonctionnement qui reflète un réglage réalisé par l'opérateur devant effectuer les soins médicaux et un réglage réalisé par le patient devant recevoir les soins médicaux, l'écran de fonctionnement étant affiché sur l'unité d'affichage de fonctionnement,
**caractérisé en ce que** la pluralité de dispositifs de soins médicaux comportent chacun un moyen de fixation de dispositif de terminal auquel le dispositif de terminal médical est fixé, et un capteur de dispositif de commande adapté pour détecter la fixation du dispositif de terminal médical,
le dispositif de soins médicaux comporte un moyen de fonctionnement côté dispositif utilisé pour faire fonctionner le dispositif et un moyen de commande côté dispositif adapté pour commander le dispositif ; et
lorsque le dispositif de terminal médical est fixé au moyen de fixation de dispositif de terminal et le capteur de dispositif de commande détecte la fixation du dispositif de terminal médical, le moyen de commande côté dispositif est adapté pour arrêter une fonction du moyen de fonctionnement côté dispositif.

2. Système médical selon la revendication 1, comprenant en outre :
un moyen de stockage d'informations d'identification d'opérateur qui stocke des informations d'identification inhérentes à l'opérateur ;
un moyen de stockage d'informations d'identification de patient qui stocke des informations d'identification inhérentes au patient ; et
un moyen de stockage d'informations d'identification de dispositif qui stocke des informations d'identification inhérentes au dispositif de soins médicaux ;
dans lequel le moyen d'identification d'opérateur, le moyen d'identification de patient et le moyen d'identification de dispositif effectuent une identification en communiquant respectivement avec le moyen de stockage d'informations d'identification d'opérateur, le moyen de stockage d'informations d'identification de patient et le moyen de stockage d'informations d'identification de dispositif.

3. Système médical selon la revendication 1 ou 2, dans lequel :
les informations d'opérateur comportent des informations de permission d'utilisation concernant le dispositif que l'opérateur est autorisé à utiliser ;
le dispositif de soins médicaux comporte un moyen de restriction d'utilisation qui fournit une commutation entre la permission et la restriction d'utilisation du dispositif sur la base des informations de permission d'utilisation qui permettent d'utiliser le dispositif ; et
le moyen de restriction d'utilisation fournit une commutation entre la permission et la restriction d'utilisation du dispositif de soins médicaux identifié par le moyen d'identification de dispositif sur la base des informations de permission d'utilisation pour l'opérateur identifié par le moyen d'identification d'opérateur.

4. Système médical selon l'une des revendications 1 à 3, dans lequel :
le dispositif de soins médicaux et le dispositif de terminal médical comportent chacun un moyen de communication sans fil ; et
les informations de fonctionnement entrées dans le dispositif de terminal médical sont transmises/reçues via le moyen de communication sans fil, de sorte que le dispositif de soins médicaux soit actionné par le dispositif de terminal médical.

5. Système médical selon l'une des revendications 1 à 4, dans lequel :
les informations de patient comportent des informations de soins médicaux concernant le patient ; et
l'unité d'affichage de fonctionnement affiche les informations de soins médicaux concernant le patient identifié par les informations d'identification de patient.

6. Dispositif de terminal médical, comprenant :
une unité d'affichage de fonctionnement adaptée pour afficher un écran de fonctionnement utilisé pour faire fonctionner un dispositif de soins médicaux ;
un moyen d'identification d'opérateur adapté pour identifier un opérateur devant effectuer des soins médicaux ;
un moyen d'identification de patient adapté pour identifier un patient devant recevoir les soins médicaux ;
un moyen d'identification de dispositif adapté pour identifier un dispositif de soins médicaux devant être utilisé pour les soins médicaux ; et
un moyen de communication adapté pour
communiquer avec un moyen de stockage d'informations qui stocke des informations de dispositifs concernant une pluralité de dispositifs de soins médicaux, des informations d'opérateurs concernant une pluralité d'opérateurs et des informations de patients concernant une pluralité de patients, et,
sur la base des informations d'opérateur correspondant à l'opérateur identifié par le moyen d'identification d'opérateur, des informations de patient correspondant au patient identifié par le moyen d'identification de patient et des informations de dispositif correspondant au dispositif de soins médicaux identifié par le moyen d'identification de dispositif, pour afficher un écran de fonctionnement sur le dispositif médical devant être utilisé pour les soins médicaux en tant qu'écran de fonctionnement qui reflète un réglage réalisé par l'opérateur devant effectuer les soins médicaux et un réglage réalisé par le patient devant recevoir les soins médicaux, l'écran de fonctionnement étant affiché sur l'unité d'affichage de fonctionnement,
**caractérisé en ce que** le dispositif de terminal médical peut être fixé au dispositif de soins médicaux afin de commander le fonctionnement du dispositif de soins médicaux, dans lequel un moyen de commande côté dispositif du dispositif de soins médicaux est adapté pour commuter une fonction d'un moyen de fonctionnement côté dispositif du dispositif de soins médicaux lors de la fixation ou du détachement du dispositif de terminal médical, et dans lequel le moyen de commande côté dispositif est adapté pour arrêter une fonction du moyen de fonctionnement côté dispositif, lorsque le dispositif de terminal médical est fixé au moyen de fixation de dispositif de terminal et un capteur de dispositif de commande du dispositif de soins médicaux détecte la fixation du dispositif de terminal médical.

7. Dispositif de terminal médical selon la revendication 6, dans lequel le moyen d'identification d'opérateur, le moyen d'identification de patient et le moyen d'identification de dispositif effectuent une identification en communiquant respectivement avec le moyen de stockage d'informations d'identification d'opérateur qui stocke des informations d'identification inhérentes à l'opérateur, le moyen de stockage d'informations d'identification de patient qui stocke des informations d'identification inhérentes au patient, et le moyen de stockage d'informations d'identification de dispositif qui stocke des informations d'identification inhérentes au dispositif de soins médicaux.

8. Dispositif de terminal médical selon la revendication 6 ou 7, comprenant en outre un moyen de communication sans fil qui communique sans fil avec un moyen de communication sans fil côté dispositif que comporte le dispositif de soins médicaux ;
dans lequel les informations de fonctionnement d'entrée sont transmises à partir du moyen de communication sans fil au moyen de communication sans fil côté dispositif, de sorte que le dispositif de terminal médical fasse fonctionner le dispositif de soins médicaux.

9. Dispositif de terminal médical selon l'une des revendications 6 à 8, dans lequel :
les informations de patient comportent des informations de soins médicaux concernant le patient ; et
l'unité d'affichage de fonctionnement affiche les informations de soins médicaux concernant le patient identifié par le moyen d'identification de patient.
